Europäisches Patentamt

European Patent Office

Office européen des brevets

(11) Publication number: **0 265 847 B1**

# EUROPEAN PATENT SPECIFICATION

(45) Date of publication of patent specification: **14.09.94**    (51) Int. Cl.⁵: **A61K 39/395**, C12P 21/00, G01N 33/577

(21) Application number: **87115515.6**

(22) Date of filing: **22.10.87**

The file contains technical information submitted after the application was filed and not included in this specification

(54) Monoclonal antibody reacting with TRF (interleukin-5).

(30) Priority: **31.10.86 JP 259957/86**

(43) Date of publication of application: **04.05.88 Bulletin 88/18**

(45) Publication of the grant of the patent: **14.09.94 Bulletin 94/37**

(84) Designated Contracting States: **CH DE FR GB LI NL SE**

(56) References cited:

**BIOLOGICAL ABSTRACT; N. HARADA et al., no. 84077206&NUM;**

**JOURNAL OF IMMUNOLOGY, vol. 125, no. 6, December 1980; TAKATSU et al., pp. 2646-2653&NUM;**

**JOURNAL OF IMMUNOLOGY, vol. 134, 1985; pp. 382-389, 3944-3951&NUM;**

**EUROPEAN JOURNAL OF IMMUNOLOGY, vol. 15, 1985; pp. 586-593&NUM;**

**NATURE, vol. 324, 1986; pp. 70-73&NUM;**

**LEXIKON DER IMMUNOLOGIE, Bundschuh-Schneeweiss-Bräuer, 1. Aufl.1988, Akademie Verlag Berlin, Medical Service München; pp. 468-469&NUM;**

(73) Proprietor: **OLYMPUS OPTICAL CO., LTD. 43-2, 2-chome, Hatagaya Shibuya-ku Tokyo 151 (JP)**

(72) Inventor: **Takatsu, Kiyoshi 301-32, Ishiwara-machi Kumamoto-shi Kumamoto-ken (JP)** Inventor: **Harada, Nobuyuki 4-54, Minami-Jutaku 4-2, Higashi-machi Kumamoto-shi Kumamoto-ken (JP)**

(74) Representative: **KUHNEN, WACKER & PARTNER Alois-Steinecker-Strasse 22 D-85354 Freising (DE)**

## Description

The present invention relates to a monoclonal antibody and, more particularly, to a monoclonal antibody which can specifically react with more than one antigenic substance, the said substance having an activity of T-cell-replacing factor (to be referred to as TRF hereinafter). The monoclonal antibody of the present invention is effective as an immunoadsorbent used for TRF purification or as a TRF-inhibitor which degrades TRF activity.

A practical application of the immunoadsorbent is in the quantitative measurement of TRF in a body fluid. The measurement of TRF is indispensable for diagnosis of autoimmune disease caused by hypersecretion of TRF or of immunodeficiency caused by insufficient secretion thereof. Another application of the immunoadsorbent is in the purification of TRF. Purified TRF obtained as a result of this application is effective as a therapeutic medicine for combatting TRF-immnodeficiency.

A practical application of the monoclonal antibody of the present invention as the TRF inhibitor degrading TRF activity is as a therapeutic medicine for the autoimmune disease caused by hypersecretion of TRF.

Furthermore, the monoclonal antibody of the present invention and the TRF purified using the antibody can serve as effective means for the purpose of studying the body's immune system.

TRF as a specific antigen to the monoclonal antibody of the present invention will be explained hereinafter.

TRF is a type of factor, called a lymphokine, secreted from T cells, and the following mechanism has been proposed as its function.

Immature B cells are bound to anti-IgM antibody at respective antigen receptor sites, and are crosslinked. As a result, the immature B cells are activated. TRF reacts with a TRF-receptor present on the surface of each activated immature B cell, thereby promoting differentiation of immature B cell to immunoglobulin-secreting-cells (Howard M., Nakanishi K. & Paul W.E., Immunol. Rev. $\underline{78}$, 185-210, 1984; Kishimoto T., Ann. Rev. Immunol., $\underline{3}$, 133-157, 1985; Dutton R.W., Folkoff R., Hirst J.A. et al., Prog. Immunol., $\underline{1}$, 355-368, 1971; Swain L.L., Howard M., Kappler J.W., et al., J. Exp. Med., $\underline{158}$, 822-835, 1983).

TRF also promotes differentiation of mature B cells producing antibodies (Takatsu K., Tominaga A. & Hamaoka T., J. Immunol., $\underline{124}$, 2414-2422, 1980; Takatsu K., Tanaka K., Tominaga A., et al., J. Immunol., $\underline{125}$, 2646-2653, 1980; Nakanishi K., et al., J. Immunol., $\underline{130}$, 2219-2224, 1983).

The TRF secreted from murine T cell hybridoma B151K12 (Takatsu K. et al., J. Immunol., $\underline{125}$, 2646-2653, 1980) has the following two characteristic biological or immunological functions.

In the case of the first function, the TRF induces IgM-secretion by $BCL_1$ leukemic B cell line (available from Texas University), and induces production of anti-dinitrophenyl antibody of IgG class in vitro by the B cells primed with dinitrophenyl-protein complex (e.g. dinitrophenyl-keyhole limpet hemocyanin (DNP-KLH), dinitrophenyl-ovalbumin (DNP-OVA)) (Takatsu K., Tanaka K., Tominaga A., et al., J. Immunol., $\underline{125}$, 2646-2653, 1980; Takatsu K., Harada N., Hara T., et al., J. Immunol., $\underline{134}$, 382-389, 1985; Harada N., Kikuchi T., Tominaga A., et al., J. Immunol., $\underline{134}$, 3944-3951, 1985).

In the case of the second function, the TRF induces proliferation of the $BCL_1$ leukemic B cell line.

In some cases, only the first function is referred to as TRF activity, the second function then being referred to as B cell growth factor II, for the purpose of distinguishing the two functions from each other. However, in this specification, the term "TRF activity" generally refers to the above-mentioned first and second functions.

Describing some physicochemical characteristics of the murine-TRF, its molecular weight falls within a range of 45,000 to 60,000 (on gel filtration), and its isoelectric point falls within a range of 4.7 to 4.9. These characteristics are different from those of 3 cell stimulatory factor 1 (BSF-1), interleukin 2 (IL-2), interleukin 3 (IL-3), and immune interferon (Takatsu K., Harada N., Hara T., et al., J. Immunol., $\underline{134}$, 382-389, 1985; Harada N., Kikuchi T., Tominaga A., et al., J. Immunol., $\underline{134}$, 3944-3951, 1985).

Note that in a recent proposal, TRF is called interleukin 5. However, the term "TRF" is used throughout this specification.

In the prior art technique, a monoclonal antibody to TRF has not yet been obtained. For this reason, purification of TRF is difficult to achieve, and the concentration of TRF in the blood of a patient suffering from autoimmune disease which appears to be caused mainly by the TRF, cannot be measured.

It is therefore an object of the present invention to provide anti-TRF monoclonal antibody.

In particular, the present invention relates to a monoclonal antibody which is capable of specifically binding with more than one antigenic substance, said antigenic substances having an activity (or function) of T-cell replacing factor [TRF (interleukin 5 {IL-5})] and being prepared from different cell lines, but does not bind with interleukin 1 (IL-1), interleukin (IL-2), interleukin (IL-3), and B-cell stimulatory factor 1 [BSF-1 (interleukin 4 {IL-4})]; and further to a monoclonal antibody which is capable of specifically binding with more than one antigenic substance, said antigenic substances having an activity (or function) of T-cell replacing factor [TRF (interleukin 5 {IL-5})] and being prepared by different methods, but does not bind with interleukin 1 (IL-1), interleukin 2 (IL-2), interleukin 3 (IL-3), and B-cell stimulatory factor 1 [BSF-1 (interleukin 4{IL-4})].

Monoclonal antibodies provided by the present invention can specifically react with the lymphokine having TRF activity and inhibit TRF activity.

The monoclonal antibody of the present invention can be produced such that fused cells between a proper type of cells immunized with TRF and another proper type of cells having a proliferation potential are cloned, and the fused cells are cultivated in vivo or in vitro. In this case, cells prepared as follows can be used as the fused cells.

For example, spleen cells obtained from a mammal preliminarily immunized with TRF, and myeloma cells of a mammal are cell-fused by means of the Koehler and Milstein cell-fusion technique (Koehler and Milstein, Nature, 256, 495-497, 1979].

The monoclonal antibody of the present invention is effective as an immunoadsorbent for TRF or as an inhibitor of TRF-activity.

This invention can be more fully understood from the following detailed description when taken in conjunction with the accompanying drawings, in which:

Figs. 1A and 1B are graphs showing a degree of inhibition with respect to TRF activities by TB13 and NC17 which are anti-TRF monoclonal antibodies;

Fig. 2 is a graph showing a degree of inhibition of r-TRF activity by NC17 which is an anti-TRF monoclonal antibody;

Fig. 3A is a photograph showing an autoradiograph of immunoprecipitates of a TRF labeled by $^{125}$I;

Fig. 3B is a graph showing measurement results of TRF activities at respective positions of a gel carrier after an SDS-PAGE corresponding to No. 1 in Fig. 3A is performed; and

Fig. 4 is a nucleotide and encoded amino-acid sequence of TRF.

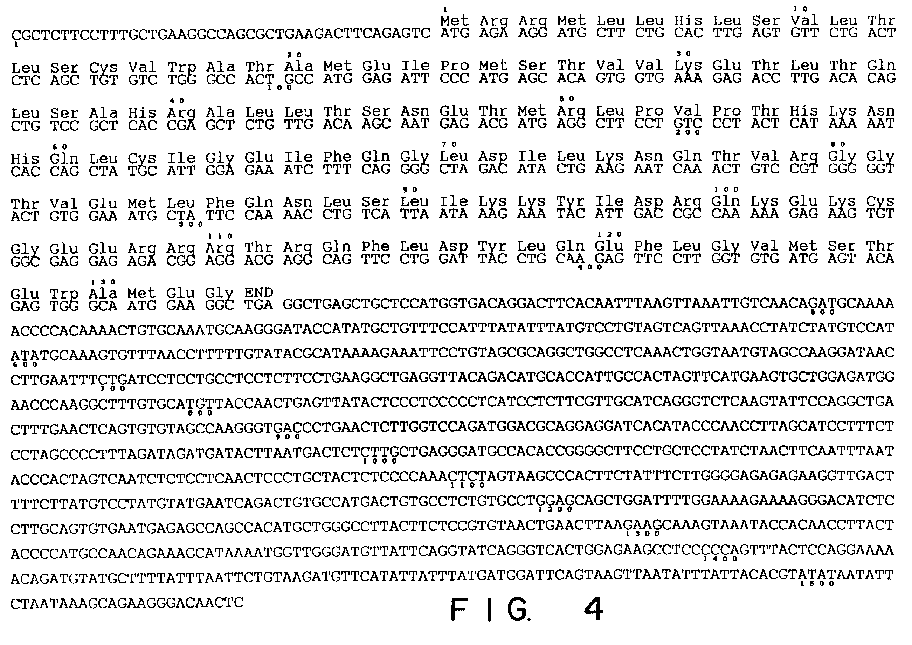

In the amino acid sequence shown in Fig. 4, the following code is used:

| | |
|---|---|
| Ala = Alanine | Leu = Leucine |
| Arg = Arginine | Lys = Lysine |
| Asn = Asparagine | Met = Methionine |
| Asp = Aspartic acid | Phe = Phenylalanine |
| Cys = Cystene | Pro = Proline |
| Gln = Glutamine | Ser = Serine |
| Glu = Glutamic acid | Thr = Threonine |
| Gly = Glycine | Trp = Tryptophan |
| His = Histidine | Tyr = Tyrosine |
| Ile = Isoleucine | Val = Valine. |

A preferred embodiment of the present invention will now be described, by way of its examples.

Example 1 (Preparation of Hybridoma Producing anti-TRF Monoclonal Antibody)

Preparation of TRF as Antigen

T cell hybridoma B151K12 cell line (Takatsu K., et al., J. Immunol., 125, 2646-2653, 1980) was cultivated in 3 ng/mℓ of 4β-phorbol-12β-myristate-12 α-acetate (available from Sigma Corp.) in serum free state for 48 hours, thus obtaining 225 ℓ of cultured supernatant containing TRF. The cultured supernatant was fractionated by ammonium sulfate precipitation in the range between 50 to 85% saturation at 4°C. The precipitate was dialyzed against 10 mM Tris-HCℓ buffer (pH 8.5) and was applied to a 22 x 450 mm DEAE-cellulose (DE52: Whatman, cilfton, NJ) column for anion exchange column choromatography that had been equilibrated in 10 mM Tris-HCℓ buffer (pH 8.5). The sample was washed into the column and was then eluted by a 0 to 0.25 M NaCℓ linear gradient in 10 mM Tris-HCℓ buffer (pH 8.5). The concentrated samples with TRF activity from DE52 column chromatography were extensively dialyzed against 50 mM Tris-HCℓ buffer (pH 8.0) and were applied on a Blue-Sepharose 4B (Pharmacia Fine Chemicals, Uppsala, Sweden) column (10 x 50 mm). Nonadsorbed fractions were collected. Nonadsorbed fractions containing TRF activity were dialyzed against 2, 2-bis (hydroxymethyl)-2, 2', 2''-nitrilotriethanol hydrochloride buffer (0.025M pH

3

EP 0 265 847 B1

6.3) and applied to a Mono P column (Pharmacia). The sample was eluted with 10% Polybuffer 74 at a pH range between 4.0 and 6.3. The sample with TRF activity from Mono P column chromatography was applied on a Superose 12 column (Pharmacia) for gel permeation and was eluted with 10 mM Hepes-buferred saline (pH 7.0). TRF active fractions from Superose 12 column chromatography were applied on a protein C4 column (Vydac, Hesperia, CA) for reverse-phase high performance liquid chromatography (HPLC) and were eluted with a linear gradient of acetonitrile (0-80%) containing 0.1% trifluoroacetic acid. Thus, partially purified TRF, a specific activity of which is increased to 1.4 million times was obtained. Note that measurement of the TRF activity in this case was conducted by the following plaque-forming cell test (PFC-test) using TRF-responding B cells.

(IgM PFC-test)

$1.5 \times 10^5$ per 0.2 mℓ per well of $BCL_1$ leukemic B cells were cultured with appropriate concentrations of test sample in microtiter plate for two days.

50 to 100 $\mu$ℓ of the suspension of cultivated $BCL_1$ leukemic B cells, 50 $\mu$ℓ of a 10% suspension of protein A coupled sheep erythrocytes, and 400 $\mu$ℓ of a 0.5% agarose solution containing an Eagle's minimum essential medium were mixed at a temperature of 45°C. The solution mixture was poured on a slide glass, and was left to stand at a room temperature for gelation. An anti mouse IgM serum was added to the resultant gel, which was incubated at a temperature of 37°C for 150 minutes. Furthermore, complement from a guinea pig was added to the resultant gel, which was incubated at a temperature of 37°C for 60 minutes. Thereafter, the number of formed plaques was counted.

(Anti-DNP IgG PFC-test)

Induction of anti-DNP IgG plaque-forming cell responses was measured as follows.

$6 \times 10^5$ per 0.2 mℓ per well of DNP-KLH primed B cells were cultured for 5 days with appropriate concentration (e.g. 1:1) of a test sample in the presence of DNP-OVA (12 ng/well). 50 to 100$\mu$ℓ of a 10% suspension of DNP-coupled sheep erythrocytes and 400 $\mu$ℓ of a 0.5% agarose solution containing an Eagle's minimum essential medium were mixed at a temperature of 45°C. The solution mixture was poured on a slide glass and was left to stand at a room temperature for gelation. Then complement from a guinea pig was added to the resultant gel. After the incubation of 37°C for 60 minutes, the number of formed plaques was counted.

Note that a unit of TRF activity in either PFC test is defined as a PFC response which is 50% of the maximal response to a standard TRF preparation. TRF activity was expressed by the number of units per culture (U/culture or PFC/culture).

Preparation of Spleen Cell Poducing Anti-TRF Antibody

A Wistar rat (femal, six weeks of age, obtained from the Shizuoka Animal Center, Hamamatsu, Japan) was immunized by $3.5 \times 10^5$ U of partially purified TRF emulsified in complete Freund's adjuvant. Furthermore, booster by $2 \times 10^5$ U of partially purified TRF was performed twice at 20-day intervals. Three days after the last booster, the spleen was taken from the Wistar rat, and the spleen cells were prepared by the following operations.

The spleen of the Wistar rat was put into 5 mℓ of Eagle's minimal essential medium, and subsequently, 4 mℓ of the above medium were injected into the spleen by an injector. After the spleen cells were minced with forceps, an operation for filling and pouring the cell suspension into and from an injection cylinder was performed three times, thereby separating the cell-mass into single cells. The obtained single cells were subjected to centrifugal separation at a speed of 1000 to 1200 rpm to be purified. The resultant single cells were suspended in 2 mℓ of the Eagle's minimal essential medium, thus obtaining a single cell suspension.

Preparation of Hybridoma Producing Anti-TRF Antiboty

Rat spleen cells producing anti-TRF antibody obtained in the above process, and mouse myelema cells P3X63-Ag8.653 (ATCC No. CRL-1580) were cell-fused by the following Koehler & Milstein's technology, thus preparing hybridoma cells producing anti-TRF antibody.

$6 \times 10^8$ spleen cells prepared in the above process and $3 \times 10^8$ mouse myeloma cells P3X63-Ag8.653 were mixed, and thereafter the cell mixture was subjected to centrifugation to form pellets. 1 mℓ of a medium (RPMI-1640, trade name; available from GIBCO LABORATORIES) containing 50% of polyethylen

4

glycol (PEG-1500, trade name; available from Wako Pure Chemical Co.) was gradually added to the pellets under stirring. Furthermore, RPMI-1640 medium was gradually added to the pellets under stirring to dilute the mixture, and thereafter, the pellets were centrifuged to remove the supernatant. The resultant pellets were suspended in medium RPMI-1640 containing 10% of fetal calf serum (FCS) to have a concentration of $1 \times 10^6$ cells/m$\ell$. The suspension was divisionally injected into a microplate having 24 wells in an amount of 1 m$\ell$/well. After 24 hr 500 $\mu\ell$ of medium were removed from each well, 500 $\mu\ell$ of Hypoxanthin-aminopterine-thymidine (HAT) medium were added to each well. Thereafter, the same procedures as above were repeated every two days, and cells were cultivated for 14 days. The same procedures as above were repeated using Hypoxanthin-thymidine (HT) medium instead of HAT medium, and cells were cultivated for 7 to 10 days. Furthermore, the cultivation was performed following the same procedures as above using RPMI-1640 medium containing 10% of FCS in place of the HT medium.

Screening of Hybridoma Producing Anti-TRF Antibody

The anti-TRF antibody of the cultured supernatant of hybridomas obtained in the above cell fusion was examined by the inhibition test of TRF activity and the absorption test of TRF activity. As a result, 61 wells of 479 wells storing hybridomas exhibited TRF activity. One well exhibiting reproducible inhibition and absorption was selected from the 61 wells, and the following cloning was performed.

Note that the inhibition test of TRF activity was performed by measurement of an amount of $^3$H-thymidine incorporation. The measurement method of the amount of $^3$H-thymidine incorporation and the method of the absorption test of the TRF activity will be described below.

(Measurement of Amount of $^3$H-thymidine Incorporation)

$1.5 \times 10^5$ BCL$_1$ leukemic B cells were cultivated in 0.2 m$\ell$ of a culture medium containing 2 units of TRF and the sample to be tested. Six hours before the completion of cultivation, 0.2 $\mu$ci of $^3$H-thymidine was added to each well. After the cells were purified by the centrifugal operation, the amount of $^3$H-thymidine incorporated into the cells was measured by liquid scintillation.

(Absorption Test of TRF Activity)

A cultured hybridoma supernatant was added to the microplate to which rabbit anti-rat Immunoglobuline antibodies were coated, and incubated at a temperature of 37°C for 4 hours. After washing, a predetermined amount of TRF was added thereto followed by incubation at a temperature of 37°C for 4 hours. Then, TRF activity remaining in solution was assessed by incubating sample with BCL$_1$ cells. The number of IgM producing cells was measured.

Cloning of the Hybridoma Cell Producing Anti-TRF Antibody

Hybridomas in the selected one well were cloned by limiting dilution analysis, thus obtaining hybridoma TB13 and hybridoma NC17.

Example 2 (Purification of Anti-TRF Monoclonal Antibody)

A purified anti-TRF monoclonal antibody was obtained from hybridoma TB13 by the following operation.

A cultured supernatant of hybridoma TB13 was applied to an affinity column filled with goat anti-rat IgG coupled agarose beads. The adsorbed fraction was eluted with a borate-buffered solution (pH =8.0) containing 3 mol (M) of potassium thiocyanate, thereby purifying anti-TRF monoclonal antibody TB13.

Mass purification was performed by the following method. $1 \times 10^7$ of TB 13 hybridomas were intraperitoneally injected into a BALB/c nu/nu mouse. About 15 to 20 days later, an ascites fluid was extracted, and was subjected to gel permeation using superose 12 column to obtain an IgG fraction, thereby obtaining purified anti-TRF monoclonal antibody TB13.

Following the same procedures as above using hybridoma NC17, purification of anti-TRF monoclonal antibody NC17 was performed.

Example 3 (Physicochemical and Immunological Properties of Anti-TRF Monoclonal Antibodies TB13 and NC17)

Immunoglobulin Class Identification of Anti-TRF Monoclonal Antibody

The immunoglobulin classes of the anti-TRF monoclonal antibodies TB13 and NC17 were identified by the Ouchterlony's method using rat typing kit 64-691-1 (Miles Scientific, USA). As a result, the class of either anti-TRF monoclonal antibody was IgG$_1$.

Molecular Weight

The molecular weights of the anti-TRF monoclonal antibodies TB13 and NC17 of IgG class were measured by the SDS-polyacrylamide gel electrophoresis in the absence of a reducing agent, and the results shown in Table 1 below were obtained.

```
                      Table 1
    Anti-TRF monoclonal antibody TB13:   158,000 to 162,000
    Anti-TRF monoclonal antibody NC17:   158,000 to 162,000
```

Immunological Specificity

In order to investigate the immunological specificity of the anti-TRF monoclonal antibody, it was examined whether or not anti-TRF monoclonal antibodies TB13 and NC17 could inhibit the activities of lymphokines of TRF, B cell stimulatory factor 1 (BSF-1), interleukin 1 (IL-1), interleukin 2 (IL-2), and interleukin 3 (IL-3). Table 2 shows the results. As can be seen from the results, both the anti-TRF monoclonal antibodies TB13 and NC17 did not inhibit the activities of BSF-1, IL-1, IL-2, and IL-3, and inhibited the activity of only TRF.

Note that in Table 2, PC61 is a rat monoclonal antibody to anti-IL 2-receptor ($\alpha$-IL-2 receptor) available from the Switzerland Cancer Laboratories (Lowenthal J.W., Zubler R.H., Nabholz M. & MacDonald R.H., Nature 315, 669-672 1985). 50 ng/m$\ell$ of PC61 inhibits proliferation of T cell line GY-1 by IL-2 (Takatsu K., Harada N., Hara T., et al., J. Immunol., 134, 382-389, 1985).

18F10 was a rat monoclonal antibody to BSF-1 of IgG$_1$ class (Ohara J. & Paul W.E., Nature 315, 333-336, 1985), which was available from the National Institutes of Health, USA. 18F10 which was diluted 30,000 times inhibited proliferation of resting B cells by BSF-1 in the presence of anti-IgM antibody.

WEHI-3 was a T-cell line producing IL-3, and was available from the Walter Eliza Hall Institute, Australia.

Examination of the specificity was performed by checking whether the respective types of monoclonal antibodies could inhibit the following activities.
* IgM secretion of BCL$_1$ leukemic B cells by TRF
* Stimulating function of resting B cells by BSF-1 + anti-IgM antibody (Ohara J. & Paul W.E., Nature 315, 333-336, 1985)
* Thymocyte stimulating function by IL-1 + PHA (phytohemagglutinin) (Mizel S.B., Immunol. Rev., 63, 51-72, 1979)
* Proliferation of T cell line GY-1 by IL-2
* Stimulation of T cell line FDC-P1 by IL-3 (Kikuchi Y., Kato R., Sano Y., Takahashi H., et al., J. Immunol., 136, 3553-3560, 1986)

6

Table 2

Response of $BCL_1$ cells to TRF[a]

| | 0 | TB13 (10 g/ml) | NC17 (10 g/ml) | PC61 (10 g/ml) | 18F10 (1:3,000) |
|---|---|---|---|---|---|
| None | 126 | 138 | 127 | - | - |
| TRF | 1,238 | 108 | 196 | 1,212 | 1,221 |

Response of B cells to anti-IgM and BSF-1[b]

| | 0 | TB13 | NC17 | PC61 | 18F10 |
|---|---|---|---|---|---|
| None | 372 | - | - | - | 238 |
| ∝IgM + BSF-1 | 7,136 | 6,832 | 6,268 | 8,362 | 138 |

Response to thymocytes to PHA and IL-1[b]

| | 0 | TB13 | NC17 | PC61 | 18F10 |
|---|---|---|---|---|---|
| None | 290 | - | - | 258 | - |
| PHA (1 l/ml) | 630 | - | - | 585 | - |
| PHA + IL-1 (2 U/ml) | 6,819 | 6,963 | 6,351 | 2,938 | 8,420 |

Response of GY-1 cells to IL-2[c]

| | 0 | TB13 | NC17 | PC61 | 18F10 |
|---|---|---|---|---|---|
| None | 248 | - | - | 238 | - |
| IL-2 (0.5 U/ml) | 18,562 | 16,385 | 14,528 | 836 | 13,826 |

Response of FDC-P1 cells to IL-3[c]

| | 0 | TB13 | NC17 | 7D4 | 18F10 |
|---|---|---|---|---|---|
| None | 1,676 | - | - | - | - |
| WEHI-3 Sup. (1:300) | 37,562 | 34,614 | 37,617 | 38,752 | - |

```
(Remarks)
        a)  The numbers of IgM PFC of BCL₁ cells in
response to TRF (2 U/mℓ) was measured at 2 days of
culture.
        b)  Uptake of ³H-thymidine was measured at 3 days
of culture and recorded as c.p.m.
        c)  Uptake of ³H-thymidine was measured at 1 day of
culture and recorded as c.p.m.
```

Activity of Anti-TRF Monoclonal Antibody

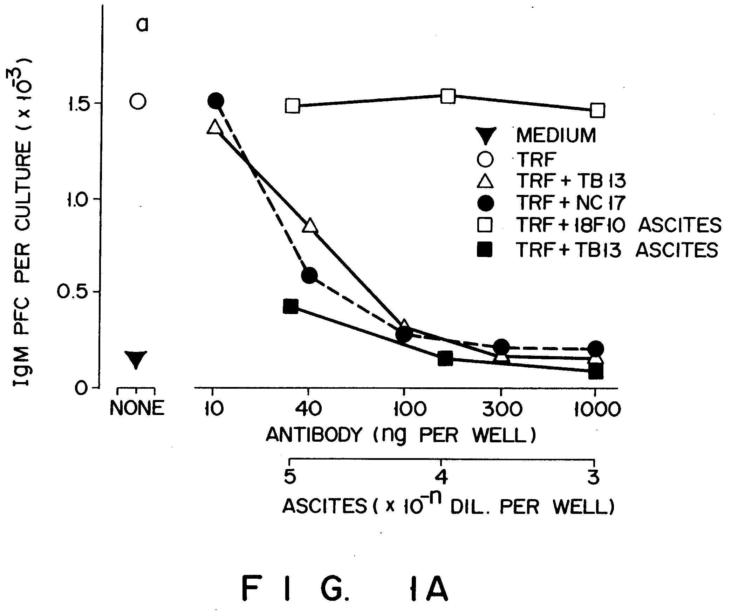

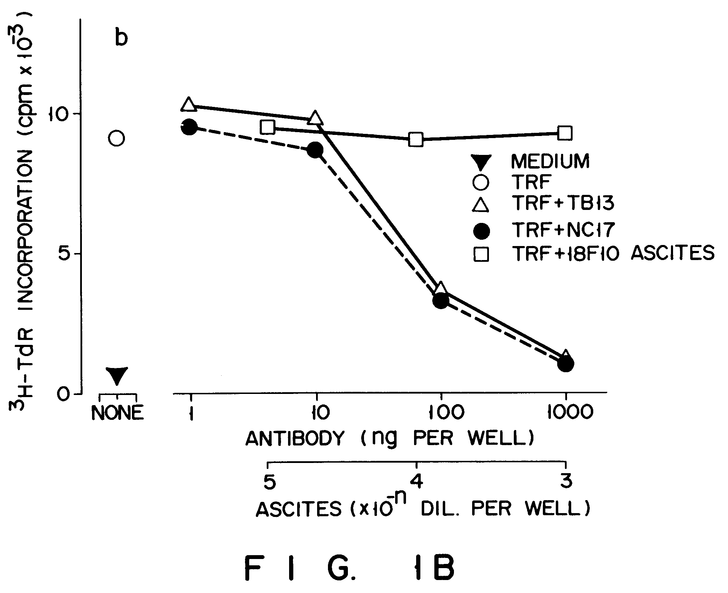

As described above, the TRF obtained from T cell hybridoma B151K12 causes $BCL_1$ leukemic B cells to be differentiated into IgM secreting cells, and accelerates proliferation of $BCL_1$ cells. The IgM PFC test and proliferation test using $BCL_1$ cells were conducted in the presence of TRF and by the anti-TRF monoclonal antibody to examine a degree of inhibition of the TRF activity by the anti-TRF monoclonal antibody. Figs. 1A and 1B respectively show the results. Note that the above tests were conducted after 1.5 x $10^5$ $BCL_1$ cells (in vivo line) per 0.2 mℓ were incubated in 200 $\mu\ell$ of a medium containing 2 U of TRF and one of three antibodies TB13, NC17, or 18F10 of various concentrations.

Fig. 1A shows the results of the number of IgM producing cells by a reversed IgM PFC assay after two days of culture. Referring to Fig. 1A, symbols indicate the following medium conditions:
▼: neither of TRF nor monoclonal antibodies was added
○: only TRF was added
△: TRF + TB13 were added
●: TRF + NC17 were added
□: TRF + 18F10 were added (ascites extracted after culture in vivo)
■: TRF + TB13 were added (ascites extracted after culture in vivo)

Fig. 1B shows the results of measurement of $^3$H-thymidine incorporation by liquid scintillation after two days of culture. Referring to Fig. 1B, symbols indicate the following medium conditions:
▼: neither of TRF nor monoclonal antibodies were added
○: only TRF was added
△: TRF + TB13 were added
●: TRF + NC17 were added
□: TRF + 18F10 were added (ascites extracted after culture in vivo)

As can be seen from the above results, the anti-TRF antibodies TB13 and NC17 strongly inhibited IgM secretion at 40 ng (Fig. 1A), and also strongly inhibited proliferation of $BCL_1$ cells. The anti-BSF-1 monoclonal antibody 18F10 of rat IgG1 class for the purpose of comparison could not inhibit neither IgM secretion nor proliferation of $BCL_1$ cells.

It was investigated whether or not the anti-TRF antibodies TB13 and NC17 could inhibit production of anti-dinitrophenyl antibody of IgG class (to be referred to as an anti-DNP IgG hereinafter) induced by the TRF. The following results were obtained.

The anti-DNP IgG PFC test was conducted for the B cells primed with DNP-protein complex. As a result, when no TRF was added, 98 PFC/culture were exhibited. When 3 U/mℓ of purified TRF were added, 638 PFC/culture were exhibited. Thus, it was demonstrated that the TRF induced production of anti-DNP IgG.

In contrast to this, when 50 ng/mℓ of anti-TRF monoclonal antibody TB13 were added at the first day of culture in addition to the TRF, the result of the same anti-DNP IgG PFC test as above was 126 PFC/culture. Thus, it was demonstrated that the anti-TRF monoclonal antibody TB13 inhibited induction of anti-DNP IgG production by TRF. The same results were obtained for the anti-TRF monoclonal antibody NC17.

The above results also revealed that the anti-TRF monoclonal antibodies TB13 and NC17 inhibited the TRF activity for B cells which were sensitized with dinitrophenyl

Example 3 (Inhibition activity of monoclonal antibodies against TRFs of Different Origins)

All TRFs used in Example 2 were produced by T cell hybridoma B151K12. Thus, it was investigated whether the anti-TRF monoclonal antibody of the present invention could inhibit the activity of TRF derived from another cell line.

Recently, the present inventors succeeded in isolation of complementary DNA (pSP6K-mTRF23) having TRF-activity. The complementary DNA was cut by restriction enzyme Sa $\ell$ I, thus obtaining a linear plasmid DNA. The linear plasmid DNA was used as a template, and m-RNA was synthesized using SP6-RNA polymerase. The resultant m-RNA was injected into Xenopus oocytes, which were incubated at a temperature of 20°C for 36 hours, thus translating PSP6K-mTRF23 by the oocytes. The nucleotide sequence of the entire cDNA was determined by the combination of the unidirectional deletion method using exonucleases III and VII and the dideoxy method (Nobuyuki Harada., Kiyoshi Takatsu., et al., Nature Vol. 324, 70-73, 1986). Nucleotide and encoded amino-acid sequence of TRF were shown in Fig. 4. A recombinant TRF (r-TRF) is a polypeptide of 133 amino acids encoded by the region extending from bases 44 through 442. The TRF product, the oocyte translation product of pSP6K-mTRF23, i.e., r-TRF had the TRF-activity for stimulating differentiation of $BCL_1$ leukemic B cells into IgM secreting cells, and enhancing proliferation of $BCL_1$ cells (Nobuyuki Harada., Kiyoshi Takatsu., et al., Nature Vol. 324, 70-73, 1986).

The following test was conducted in order to examine the inhibiting effect of anti-TRF monoclonal antibody NC17 for the TRF-activity of the r-TRF.

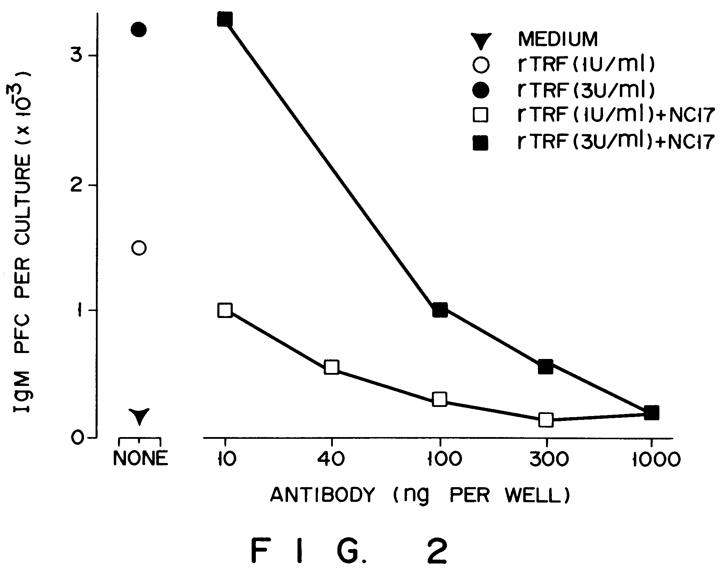

In the presence or absence of the anti-TRF monoclonal antibody NC17 but in the presence of the r-TRF, $1.5 \times 10^5/0.2$ m $\ell$ of $BCL_1$ leukemic B cells were cultivated. After two days of culture, the IgM PFC test using protein A coupled sheep erythrocytes was conducted, and the number of IgM secreting cells was counted. Fig. 2 shows the results.

The graph of Fig. 2 shows a state wherein the anti-TRF monoclonal antibody NC17 inhibits the TRF activity of the r-TRF in accordance with its addition concentration. Referring to Fig. 2, symbols indicate the following culture conditions:

▼: neither of r-TRF nor monoclonal antibody NC17 were added

○: only r-TRF was added (1 U/m $\ell$)

●: only r-TRF was added (3 U/m $\ell$)

□: r-TRF (1 U/m $\ell$) + NC17 were added

■: r-TRF (3 U/m $\ell$) + NC17 were added

As can be seen from the results shown in Fig. 2, the anti-TRF monoclonal antibody NC17 inhibits the TRF activity of the r-TRF depending on an amount of addition.

Similarly, it was examined whether the anti-TRF antibody NC17 could inhibit secretion of anti-DNP IgG induced by the r-TRF.

The anti-DNP IgG PFC test was performed for the DNP primed B cells. As a result, when no TRF was added, 169 PFC/culture were exhibited. When 3 U/m $\ell$ of r-TRF were added, 1003 PFC/culture was exhibited. Thus, it was demonstrated that the r-TRF induces production of anti-DNP IgG.

In contrast to this, when 1 $\mu$g/m $\ell$ of anti-TRF monoclonal antibody NC17 were added at the first day of culture in addition to the r-TRF, the result of the anti-DNP IgG PFC test was 328 PFC/culture. Thus, it was demonstrated that the anti-TRF monoclonal antibody NC17 could inhibit induction of anti-DNP IgG production.

The results of Example 3 reveal that the anti-TRF monoclonal antibody of the present invention specifically reacts with not only to the TRF having a specific origin but also to TRFs having various origins, and that the monoclonal antibody can inhibit its TRF-activities with high reproducibility.

Example 4 (Application of Anti-TRF Monoclonal Antibody)

Application in Immunoadsorbent

2.9 mg of a mixture of anti-TRF antibodies TB13 and NC17 were conjugated with 3 g of Sepharose CL-4B (Pharmacia) activated with cyanogen bromide, and the conjugate was used as an immunoadsorbent.

10 m $\ell$ of cultured supernatant of T cell hybridoma B151K12 which was partially purified by gel filtration with reverse phase HPLC were used as a sample, and was supplied in an affinity column filled with the immunoadsorbent. Then, an effluent liquid (10 m $\ell$) was collected. The affinity column was sequentially washed with 40 m $\ell$ of 1M NaC $\ell$ solution, 40 m $\ell$ of 0.5% nonidet P-40 (NP-40), 40 m $\ell$ of phosphate-buffered saline, and 40 m $\ell$ of water. Thereafter, 8 m $\ell$ of 0.8M acetic acid solution were flowed to elute the TRF trapped in the affinity column. The eluate was freeze-dried, and was dissolved again in 0.8 m $\ell$ of

water.

The TRF activities of the sample, the effluent liquid, and the eluate were measured by the IgM PFC-test using BCL$_1$ leukemic B cells. Table 3 shows the results.

### Table 3

| | Amount (ml) | TRF-activity | |
|---|---|---|---|
| | | U/ml | Total (U) |
| Sample | 10 | 1,200 | 12,000 |
| Effluent liquid | 10 | 13.8 | 138 |
| Eluate (0.8M Acetic Acid) | 0.8 | 13,500 | 10,800 |

As can be seen from the above results, of total TRF-activity 12,000 U of the sample, only 138 U were flowed out, and the effluent contained 10,800 U of TRF. As a result, the degree of purification of the sample was only 1,200 U/ml, while the degree of purification of the effluent liquid could be remarkably improved to 13,500 U/ml.

For the sake of comparison, the same test as above was conducted using normal rat Ig coupled to Sepharose CL-4B as an immunoadsorbent. All the TRFs were flowed out in this case.

Application in Elucidation of Physicochemical Characteristics of TRF

$1 \times 10^5$ U of purified TRF produced by hybridoma B151K12 were labeled by $^{125}$I using the chrolamin-T method. After unbound $^{125}$I was removed using Sephadex G-25 (Pharmacia), the $^{125}$I-labeled TRF was subjected to immunoprecipitated Sepharose CL4B column to which anti-TRF monoclonal antibody TB13 had been coupled. $^{125}$I-labeled TRF was eluted with a buffer solution containing 2% sodium dodecyl sulfate (SDS) in the presence or absence of 2-mercaptoethanol. For the eluted fraction, SDS-polyacrylamide gel electrophoresis (SDS-PAGE) was performed, and the $^{125}$I-labeled TRF was detected by autoradiography. For comparison, normal rat immunoglobulin coupled Sepharose CL4B column was used in place of anti-TRF monoclonal antibody TB13 coupled Sepharose CL-4B column, and the test was conducted following the same procedures as above.

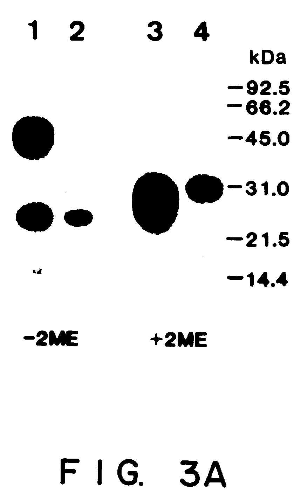

Fig. 3A shows the test results.

Referring to Fig. 3A, No. 1 represents the result of the Example in the absence of 2-mercaptoethanol (-2ME), No. 2 represents the result of control under -2ME, No. 3 represents the result of the Example in the presence of 2-mercaptoethanol (+2ME), and No. 4 represents the result of control under +2ME.

As can be understood from the results Nos. 1 and 2 in Fig. 3A, under a non-reducing condition containing no 2-mercaptoethanol, the molecular weight of the main fraction was 44,000 to 48,000. As can be seen from collation with the results Nos. 3 and 4 in Fig. 3A, the main fraction (molecular weight = 44,000 to 48,000) under the non-reducing condition was drifted to the fraction having a molecular weight of 23,000 to 26,000 under the reducing condition containing 2-mercaptoethanol. It can be considered from this fact that the TRF consists of two subunits having a molecular weight of 23,000 to 26,000.

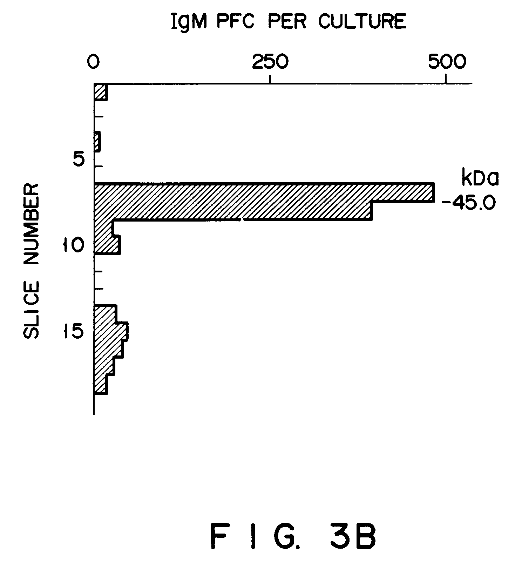

After SDS-PAGE of No. 1 in Fig. 3A was performed, the polyacrylamide gel was sliced along the direction of electrophoresis at every 2 mm. A substance contained in each slice was electrophoretically eluted, and the TRF-activity of each elution was measured by the IgM PFC test. Fig. 3B shows the test results.

The results shown in Fig. 3B reveal that the fraction having a molecular weight of 44,000 to 48,000 (in particular, about 46,000) exhibited strong TRF-activity. Thus, it could be demonstrated that the anti-TRF monoclonal antibody TB13 was specifically bounded to a protein having the TRF-activity and a molecular weight of 44,000 to 48,000.

The test results shown in Fig. 1 and Table 2 previously also show the relationship between TRF and some lymphokines. More specifically, the activity of TRF was considerably inhibited by 50 ng/ml of anti-TRF monoclonal antibody TB13 or NC17, while lymphokines of IL-1, IL-2, IL-3, and BSF-1 could not be

inhibited by 10μg/mℓ of anti-TRF monoclonal antibody TB13 or NC17.

As is understood from the above description, TRF was a quite different lymphokine from BSF-1 IL-1, IL-2, and IL-3. This shows that TRF was a unique lymphokine together with a report teaching that TRF preparation includes no interferon activity (Takatsu K., et al., J. Immunol., 134, 382-389, 1985).

As has been described above, the monoclonal antibody of the present invention can react with all the substances having TRF activity. Therefore, the monoclonal antibody of the present invention is effective for purification of TRF and as a means for examining the molecular characteristics of TRF.

Since the monoclonal antibody of the present invention inhibits highly TRF-activity, investigation of a wide variety of immune responses in vitro and in vivo can be allowed, and it can be expected that the function of the TRF is identified.

**Claims**

1. A monoclonal antibody which is capable of specifically binding with more than one antigenic substance, said antigenic substances having an activity (or function) of T-cell replacing factor [TRF (interleukin 5 {IL-5})] and being prepared from different cell lines, but does not bind with interleukin 1 (IL-1), interleukin 2 (IL-2), interleukin 3 (IL-3), and B-cell stimulatory factor 11 [BSF-1 (interleukin 4 {IL-4})].

2. A monoclonal antibody which is capable of specifically binding with more than one antigenic substance, said antigenic substances having an activity (or function) of T-cell replacing factor [TRF (interleukin 5 {IL-5})] and being prepared by different methods, but does not bind with interleukin 1 (IL-1), interleukin 2 (IL-2), interleukin 3 (IL-3), and B-cell stimulatory factor 1 [BSF-1 (interleukin 4 {IL-4})].

3. A monoclonal antibody according to claims 1 or 2, characterized in that said monoclonal antibody inhibits TRF-activity of said antigenic substance.

4. A monoclonal antibody according to claim 1 or 2, characterized in that said antigenic substance having TRF-activity comprises a peptide containing the following 133 amino acid sequence, taken from amino-terminus to carboxy-terminus:

```
Met Arg Arg Met Leu Leu His Leu Ser Val Leu Thr Leu Ser
Cys Val Trp Ala Thr Ala Met Glu Ile Pro Met Ser Thr Val
Val Lys Glu Thr Leu Thr Gln Leu Ser Ala His Arg Ala Leu
Leu Thr Ser Asn Glu Thr Met Arg Leu Pro Val Pro Thr His
Lys Asn His Gln Leu Cys Ile Gly Glu Ile Phe Gln Gly Leu
Asp Ile Leu Lys Asn Gln Thr Val Arg Gly Gly Thr Val Glu
Met Leu Phe Gln Asn Leu Ser Leu Ile Lys Lys Tyr Ile Asp
Arg Gln Lys Glu Lys Cys Gly Glu Glu Arg Arg Arg Thr Arg
Gln Phe Leu Asp Tyr Leu Gln Glu Phe Leu Gly Val Met Ser
Thr Glu Trp Ala Met Glu Gly.
```

5. A monoclonal antibody according to claim 4, characterized in that said TRF is a recombinant TRF.

6. A monoclonal antibody according to claim 4, characterized in that said TRF is secreted from T cells.

7. A monoclonal antibody according to claim 1, characterized in that an immunoglobulin class is IgG$_1$.

8. A monoclonal antibody according to claim 1, characterized in that said monoclonal antibody inhibits TRF-activity for proliferating and differentiating activated immature B cells primed with antigen into antibody secreting cells.

9. A monoclonal antibody according to claim 8, characterized in that said monoclonal antibody does not inhibit activities of IL-1, IL-2, IL-3, and BSF-1.

10. A monoclonal antibody according to claim 2, characterized in that said monoclonal antibody is produced such that fused cells between a proper type of cells immunized with TRF and another proper type of cells having large proliferation potential are cloned, and the fused cells are cultivated in vivo or in vitro.

11. A monoclonal antibody according to claim 10, characterized in that the fused cells are hybridoma obtained by cell-fusing spleen cells extracted from a mammal immunized with the TRF and mouse myeloma cells.

12. A monoclonal antibody according to claim 11, characterized in that said spleen cells are obtained from a rat.

13. A monoclonal antibody according to claim 7, characterized in that said monoclonal antibody inhibits TRF-activity for proliferation of $BCL_1$ leukemic B cell line.

14. A monoclonal antibody according to claim 7, characterized in that said monoclonal antibody inhibits TRF-activity for differentiating B cells primed with dinitrophenyl complex into anti-dinitrophenyl antibody of class IgG secreting cells.

15. A monoclonal antibody according to claim 12, characterized in that the ability to inhibit TRF-activity is confirmed by IgM PFC test.

16. A monoclonal antibody according to claim 14, characterized in that the ability to inhibit TRF-activity is confirmed by IgG PFC test.

17. A monoclonal antibody according to claim 13, characterized in that the ability to inhibit TRF-activity is confirmed by decrease in thymidine incorporation.

18. A monoclonal antibody according to claim 1 or 2, characterized in that said monoclonal antibody is coupled to an insoluble material for detection of TRF in a body fluid or purification of TRF.

## Patentansprüche

1. Monoklonaler Antikörper, der zur spezifischen Bindung an mehr als eine antigene Substanz fähig ist, wobei die antigenen Substanzen eine Aktivität (oder Funktion) des T-Zell-ersetzenden Faktors [T-cell replacing factor, TRF (Interleukin 5 {IL-5})] aufweisen und aus verschiedenen Zellinien hergestellt werden, aber nicht an Interleukin 1 (IL-1), Interleukin 2 (IL-2), Interleukin 3 (IL-3) und den B-Zell-stimulierenden Faktor 1 [BSF-1 (Interleukin 4 {IL-4})] bindet.

2. Monoklonaler Antikörper, der zur spezifischen Bindung an mehr als eine antigene Substanz fähig ist, wobei die antigenen Substanzen eine Aktivität (oder Funktion) des T-Zell-ersetzenden Faktors [T-cell-replacing factor, TRF (Interleukin 5 {IL-5})] aufweisen und nach verschiedenen Verfahren hergestellt werden, aber nicht an Interleukin 1 (IL-1), Interleukin 2 (IL-2), Interleukin 3 (IL-3) und den B-Zell-stimulierenden Faktor 1 [BSF-1 (Interleukin 4 {IL-4})] bindet.

3. Monoklonaler Antikörper nach Anspruch 1 oder 2, dadurch gekennzeichnet, daß der monoklonale Antikörper die TRF-Aktivität der antigenen Substanz hemmt.

4. Monoklonaler Antikörper nach Anspruch 1 oder 2, dadurch gekennzeichnet, daß die antigene Substanz, die TRF-Aktivität aufweist, ein Peptid umfaßt, das - vom Amino-Ende zum Carboxy-Ende gelesen - die folgende Sequenz aus 133 Aminosäuren enthält:

```
Met Arg Arg Met Leu Leu His Leu Ser Val Leu Thr Leu Ser
Cys Val Trp Ala Thr Ala Met Glu Ile Pro Met Ser Thr Val
Val Lys Glu Thr Leu Thr Gln Leu Ser Ala His Arg Ala Leu
Leu Thr Ser Asn Glu Thr Met Arg Leu Pro Val Pro Thr His
Lys Asn His Gln Leu Cys Ile Gly Glu Ile Phe Gln Gly Leu
Asp Ile Leu Lys Asn Gln Thr Val Arg Gly Gly Thr Val Glu
Met Leu Phe Gln Asn Leu Ser Leu Ile Lys Lys Tyr Ile Asp
Arg Gln Lys Glu Lys Cys Gly Glu Glu Arg Arg Arg Thr Arg
Gln Phe Leu Asp Tyr Leu Gln Glu Phe Leu Gly Val Met Ser
Thr Glu Trp Ala Met Glu Gly.
```

**5.** Monoklonaler Antikörper nach Anspruch 4, dadurch gekennzeichnet, daß TRF ein rekombinantes TRF ist.

**6.** Monoklonaler Antikörper nach Anspruch 4, dadurch gekennzeichnet, daß TRF von T-Zellen sezerniert wird.

**7.** Monoklonaler Antikörper nach Anspruch 1, dadurch gekennzeichnet, daß eine Immunglobulinklasse IgG$_1$ ist.

**8.** Monoklonaler Antikörper nach Anspruch 1, dadurch gekennzeichnet, daß der monoklonale Antikörper die TRF-Aktivität, aktivierte unreife B-Zellen, die mit Antigen zu Antikörper-sezernierenden Zellen angeregt wurden, zu proliferieren und zu differenzieren, hemmt.

**9.** Monoklonaler Antikörper nach Anspruch 8, dadurch gekennzeichnet, daß der monoklonale Antikörper keine IL-1-, IL-2-, IL-3- und BSF-1-Aktivitäten hemmt.

**10.** Monoklonaler Antikörper nach Anspruch 2, dadurch gekennzeichnet, daß der monoklonale Antikörper derartig hergestellt wird, daß Zellen, die aus einem geeigneten mit TRF immunisierten Zelltyp und einem anderen geeigneten Zelltyp mit großem Proliferationspotential fusioniert wurden, geklont werden, und die fusionierten Zellen in vivo oder in vitro gezüchtet werden.

**11.** Monoklonaler Antikörper nach Anspruch 10, dadurch gekennzeichnet, daß die fusionierten Zellen Hybridome sind, die durch Zellfusion von Milzzellen, die aus einem Säuger gewonnen wurden, der mit TRF immunisiert wurde, und Maus-Myelomzellen erhalten wurden.

**12.** Monoklonaler Antikörper nach Anspruch 11, dadurch gekennzeichnet, daß die Milzzellen aus Ratten erhalten wurden.

**13.** Monoklonaler Antikörper nach Anspruch 7, dadurch gekennzeichnet, daß der monoklonale Antikörper die TRF-Aktivität, die BCL$_1$-Leukämie-B-Zellinie zu proliferieren, hemmt.

**14.** Monoklonaler Antikörper nach Anspruch 7, dadurch gekennzeichnet, daß der monoklonale Antikörper die TRF-Aktivität hemmt, B-Zellen zu differenzieren, die mit Dinitrophenyl-Komplex zu Zellen angeregt wurden, die Anti-Dinitrophenyl-Antikörper der Klasse IgG sezernieren.

**15.** Monoklonaler Antikörper nach Anspruch 12, dadurch gekennzeichnet, daß die Fähigkeit, TRF-Aktivität zu hemmen, durch den IgM-PFC-Test bestätigt wird.

**16.** Monoklonaler Antikörper nach Anspruch 14, dadurch gekennzeichnet, daß die Fähigkeit, TRF-Aktivität zu hemmen, durch den IgG-PFC-Test bestätigt wird.

**17.** Monoklonaler Antikörper nach Anspruch 13, dadurch gekennzeichnet, daß die Fähigkeit, TRF-Aktivität zu hemmen, durch die Abnahme des Thymidin-Einbaus bestätigt wird.

**18.** Monoklonaler Antikörper nach Anspruch 1 oder 2, dadurch gekennzeichnet, daß der monoklonale Antikörper zur Feststellung von TRF in Körperflüssigkeit oder zur Reinigung von TRF an ein unlösliches Material gekoppelt ist.

**Revendications**

**1.** Anticorps monoclonal qui est capable de se lier spécifiquement à plus d'une substance antigénique, lesdites substances antigéniques présentant une activité ou une fonction de facteur de remplacement des cellules T (TRF, interleukine 5 ou IL-5) et étant préparées à partir de différentes lignées cellulaires, mais qui ne se lie ni à l'interleukine 1 (IL-1),ni à l'interleukine 2 (IL-2), ni à l'interleukine 3 (IL-3), ni au facteur de stimulation des cellules B (BSF-1, interleukine 4 ou IL-4).

**2.** Anticorps monoclonal qui est capable de se lier spécifiquement à plus d'une substance antigénique, lesdites substances antigéniques présentant une activité ou une fonction de facteur de remplacement des cellules T (TRF interleukine 5 ou IL-5) et étant préparées selon différents procédés, mais qui ne se lie ni à l'interleukine 1 (IL-1), ni à l'interleukine 2 (IL-2), ni à l'interleukine 3 (IL-3), ni au facteur de stimulation des cellules B (BSF-1,interleukine 4 ou IL-4).

**3.** Anticorps monoclonal conforme à la revendication 1 ou 2, caractérisé en ce que ledit anticorps monoclonal inhibe l'activité de TRF de ladite substance antigénique.

**4.** Anticorps monoclonal conforme à la revendication 1 ou 2, caractérisé en ce que ladite substance antigénique présentant une activité de TRF comprend un peptide qui contient la séquence suivante de 133 acides aminés, de l'extrémité amino-terminale à l'extrémité carboxy-terminale :

```
Met Arg Arg Met Leu Leu His Leu Ser Val Leu Thr Leu Ser
Cys Val Trp Ala Thr Ala Met Glu Ile Pro Met Ser Thr Val
Val Lys Glu Thr Leu Thr Gln Leu Ser Ala His Arg Ala Leu
Leu Thr Ser Asn Glu Thr Met Arg Leu Pro Val Pro Thr His
Lys Asn His Gln Leu Cys Ile Gly Glu Ile Phe Gln Gly Leu
Asp Ile Leu Lys Asn Gln Thr Val Arg Gly Gly Thr Val Glu
Met Leu Phe Gln Asn Leu Ser Leu Ile Lys Lys Tyr Ile Asp
Arg Gln Lys Glu Lys Cys Gly Glu Glu Arg Arg Arg Thr Arg
Gln Phe Leu Asp Tyr Leu Gln Glu Phe Leu Gly Val Met Ser
Thr Glu Trp Ala Met Glu Gly.
```

**5.** Anticorps monoclonal conforme à la revendication 4, caractérisé en ce que ledit TRF est un TRF recombinant.

**6.** Anticorps monoclonal conforme à la revendication 4, caractérisé en ce que ledit TRF est sécrété par des cellules T.

**7.** Anticorps monoclonal conforme à la revendication 1, caractérisé en ce qu'il s'agit d'une immunoglobuline de la classe $IgG_1$.

**8.** Anticorps monoclonal conforme à la revendication 1, caractérisé en ce que ledit anticorps monoclonal inhibe l'activité de TRF qui provoque la prolifération de cellules B immatures activées et la différentiation de telles cellules, sensibilisées avec un antigène, en cellules sécrétant des anticorps.

14

9. Anticorps monoclonal conforme à la revendication 8, caractérisé en ce que ledit anticorps monoclonal n'inhibe pas les activités d'IL-1, d'IL-2, d'IL-3 et de BSF-1.

10. Anticorps monoclonal conforme à la revendication 2, caractérisé en ce que l'on produit ledit anticorps monoclonal en clonant des cellules résultant de la fusion de cellules de type approprié, immunisées avec du TRF, et de cellules d'un autre type approprié, possédant un grand potentiel de prolifération, et en cultivant ces cellules fusionnées, in vivo ou in vitro.

11. Anticorps monoclonal conforme à la revendication 10, caractérisé en ce que les cellules fusionnées sont des hybridomes obtenus par fusion cellulaire de splénocytes extraits d'un mammifère immunisé avec du TRF et de cellules de myélome de souris.

12. Anticorps monoclonal conforme à la revendication 11, caractérisé en ce que lesdits splénocytes proviennent d'un rat.

13. Anticorps monoclonal conforme à la revendication 7, caractérisé en ce que ledit anticorps monoclonal inhibe l'activité de TRF qui provoque la prolifération de la lignée $BCL_1$ de cellules B leucémiques.

14. Anticorps monoclonal conforme à la revendication 7, caractérisé en ce que ledit anticorps monoclonal inhibe l'activité de TRF qui provoque la différentiation de cellules B, sensibilisées avec un complexe de dinitrophényle, en cellules sécrétant un anticorps anti-dinitrophényle de la classe des IgG.

15. Anticorps monoclonal conforme à la revendication 12, caractérisé en ce que sa capacité à inhiber l'activité de TRF est confirmée par un test PFC en présence d'IgM.

16. Anticorps monoclonal conforme à la revendication 14, caractérisé en ce que sa capacité à inhiber l'activité de TRF est confirmée par un test PFC en présence d'IgG.

17. Anticorps monoclonal conforme à la revendication 13, caractérisé en ce que sa capacité à inhiber l'activité de TRF est confirmée par une baisse de l'incorporation de thymidine.

18. Anticorps monoclonal conforme à la revendication 1 ou 2, caractérisé en ce que ledit anticorps monoclonal est couplé à une substance insoluble, pour une opération de détection de TRF dans un fluide corporel ou de purification de TRF.

FIG. IA

F I G. I B

F I G.  2

F I G. 3A

F I G. 3B

EP 0 265 847 B1

```
                                                      1                                          10
                                                     Met Arg Arg Met Leu Leu His Leu Ser Val Leu Thr
CGCTCTTCCTTTGCTGAAGGCCAGCGCTGAAGACTTCAGAGTC          ATG AGA AGG ATG CTT CTG CAC TTG AGT GTT CTG ACT

                        20                                                    30
Leu Ser Cys Val Trp Ala Thr Ala Met Glu Ile Pro Met Ser Thr Val Val Lys Glu Thr Leu Thr Gln
CTC AGC TGT GTC TGG GCC ACT GCC ATG GAG ATT CCC ATG AGC ACA GTG GTG AAA GAG ACC TTG ACA CAG

            40                                        50
Leu Ser Ala His Arg Ala Leu Leu Thr Ser Asn Glu Thr Met Arg Leu Pro Val Pro Thr His Lys Asn
CTG TCC GCT CAC CGA GCT CTG TTG ACA AGC AAT GAG ACG ATG AGG CTT CCT GTC CCT ACT CAT AAA AAT

    60                                    70                                              80
His Gln Leu Cys Ile Gly Glu Ile Phe Gln Gly Leu Asp Ile Leu Lys Asn Gln Thr Val Arg Gly Gly
CAC CAG CTA TGC ATT GGA GAA ATC TTT CAG GGG CTA GAC ATA CTG AAG AAT CAA ACT GTC CGT GGG GGT

                        90                                    100
Thr Val Glu Met Leu Phe Gln Asn Leu Ser Leu Ile Lys Lys Tyr Ile Asp Arg Gln Lys Glu Lys Cys
ACT GTG GAA ATG CTA TTC CAA AAC CTG TCA TTA ATA AAG AAA TAC ATT GAC CGC CAA AAA GAG AAG TGT

                110                                       120
Gly Glu Glu Arg Arg Arg Thr Arg Gln Phe Leu Asp Tyr Leu Gln Glu Phe Leu Gly Val Met Ser Thr
GGC GAG GAG AGA CGG AGG ACG AGG CAG TTC CTG GAT TAC CTG CAA GAG TTC CTT GGT GTG ATG AGT ACA

            130
Glu Trp Ala Met Glu Gly END
GAG TGG GCA ATG GAA GGC TGA GGCTGAGCTGCTCCATGGTGACAGGACTTCACAATTTAAGTTAAATTGTCAACAGATGCAAAA

ACCCCACAAAACTGTGCAAATGCAAGGGATACCATATGCTGTTTCCATTTATATTTATGTCCTGTAGTCAGTTAAACCTATCTATGTCCAT

ATATGCAAAGTGTTTAACCTTTTTGTATACGCATAAAAGAAATTCCTGTAGCGCAGGCTGGCCTCAAACTGGTAATGTAGCCAAGGATAAC

CTTGAATTTCTGATCCTCCTGCCTCCTCTTCCTGAAGGCTGAGGTTACAGACATGCACCATTGCCACTAGTTCATGAAGTGCTGGAGATGG

AACCCAAGGCTTTGTGCATGTTACCAACTGAGTTATACTCCCTCCCCCTCATCCTCTTCGTTGCATCAGGGTCTCAAGTATTCCAGGCTGA

CTTTGAACTCAGTGTGTAGCCAAGGGTGACCCTGAACTCTTGGTCCAGATGGACGCAGGAGGATCACATACCCAACCTTAGCATCCTTTCT

CCTAGCCCCTTTAGATAGATGATACTTAATGACTCTCTTGCTGAGGGATGCCACACCGGGGCTTCCTGCTCCTATCTAACTTCAATTTAAT

ACCCACTAGTCAATCTCTCCTCAACTCCCTGCTACTCTCCCCAAACTCTAGTAAGCCCACTTCTATTTCTTGGGGAGAGAGAAGGTTGACT

TTTCTTATGTCCTATGTATGAATCAGACTGTGCCATGACTGTGCCTCTGTGCCTGGAGCAGCTGGATTTTGGAAAAGAAAAGGGACATCTC

CTTGCAGTGTGAATGAGAGCCAGCCACATGCTGGGCCTTACTTCTCCGTGTAACTGAACTTAAGAAGCAAAGTAAATACCACAACCTTACT

ACCCCATGCCAACAGAAAGCATAAAATGGTTGGGATGTTATTCAGGTATCAGGGTCACTGGAGAAGCCTCCCCCAGTTTACTCCAGGAAAA

ACAGATGTATGCTTTTATTTAATTCTGTAAGATGTTCATATTATTTATGATGGATTCAGTAAGTTAATATTTATTACACGTATATAATATT

CTAATAAAGCAGAAGGGACAACTC
```

# FIG. 4